# EUROPEAN PATENT APPLICATION

(11) **EP 1 967 222 A2**
(43) Date of publication of application: **10.09.2008**
(21) Application number: 08009230.7
(22) Date of filing: 13.01.2000
(51) Int. Cl.: A61M 1/36, A61M 5/142

(54) **Multipurpose machine for monitoring and control of therapies**

(30) Priority: 16.11.1999 IT MO20000259
(62) Divisional of application: 00100189.0
(71) Applicant: Rand S.r.l., 41036 Medolla (MO) (IT)
(72) Inventor: Benatti, Luigi, 41037 Mirandola (MO) (IT)
(74) Representative: Feltrinelli, Secondo Andrea

(57) **Abstract**

The multipurpose machine for monitoring an control of therapies comprises a head (2) which has a box-like structure, a base (3) to support said head (2) and modifying means (4) for modifying its dimensions.

## Description

### FIELD OF THE INVENTION.

The present invention is a divisional application of the parent European Application No.: 00100189.1 filed on 13.01.2000 and relates to a multipurpose machine, for monitoring and control of therapies, particularly regional cancer therapies in oncology.

### STATE OF THE ART.

Regional cancer therapies currently applied in oncology use techniques which provide for the surgical or radiological insertion of special vascular catheters with two or three passages, in order to reach and directly perfuse the tumour or the body region invaded by the neoplasia.

This kind of machines are normally utilized in operating theatres wherein a great number of further machines of many kinds are present, and among which surgeons and medical staff have to move easily during their daily work.

It is therefore very important that such a multipurpose machines may have the smaller dimension as possible, in connection with performances to be achieved and components, in order to facilitate movements of medical and paramedical staff inside the operating theatre during their use for carrying out therapies on a patient and not interfere after use has been finished.

Known multipurpose machines are provided with a head with a box-like structure, inside which are accommodated one or more sets of duct circuits, which are connectable one another alternatively, in order to carry out therapies, e.g. the infusion of chemotherapeutics agents to a patient and to perform intraperitoneal hyperthermic perfusion therapies.

Along said circuits are also arranged respective pumping units, to draw blood to be treated from a patient, to circulate along the ducts and to return to the patient after treatment of blood have been finished.

Furthermore, ducts are provided for, to connect the patient to the multipurpose machine, namely a drawing duct and a return duct.

According to prior art, e.g. EP 0829265, the head is arranged on a base, generally provided with wheels, and is fitted and blocked on the top of a vertical bearing, namely at least an upright, which arises from the base, up to an height suitable for allowing the head to be substantially aligned with the rest height of the patient when he is laying down on a bed.

This know machines have some drawbacks.

A first drawback is that their overall dimensions cannot be modified and, therefore, owing to their size, both when they are in use and when are out of service and switched off, they can interfere with movements of medical and paramedical staff inside the operating theatre during operations.

Another drawback is that know machines are movable on the floor, but they cannot be portable, e.g. loaded on a car or an ambulance, because their dimensions (their height, especially), prevents from being loaded on and/or unloaded easily.

### OBJECTS OF THE INVENTION.

An object of the invention is to improve the technological background.

Another object of the invention is to provide medical and paramedical staff with a multipurpose machine for monitoring and control of therapies, in particular both hypoxic perfusion and intraperitoneal hyperthermal perfusion therapies having a fully automated procedure and maximum safety for the patient.

A further object of the invention is to provide for a multipurpose machine for monitoring and control of therapies, which can have an extremely compact structure, if necessary, and that is easy to carry and absolutely reliable in operation.

Another object of the invention is to get ready a multipurpose machine for monitoring and control of therapies that can be portable.

Another object of the invention is to provide a multipurpose machine for monitoring and control of therapies that can be modified in its dimensions, especially for being adaptable to the height of an operating table and/or of a bed on which a patient lies.

According to a first aspect of the invention, it is provided a multipurpose machine for monitoring and control of therapies comprising: a head which has a box-like structure; a base to support said head, characterized in that modifying means for modifying its dimensions are provided for.

The multipurpose machine for monitoring and control of therapies allows, therefore, to modify its dimensions, so that it can be made portable and/or adaptable to available spaces in which it is to be placed and to the height of a bed or of an operating table.

### BRIEF DESCRIPTION OF THE DRAWINGS.

Further characteristics and advantages will become apparent from the following description of a preferred embodiment of a multipurpose machine for monitoring and control of therapies in oncology, illustrated only by way of non-limitative example in the accompanying drawings, wherein:
Figure 1 is a front view of the machine according to the invention in a portable compact configuration;
Figure 2 is a corresponding front view of the machine in an extended possible configuration for use;
Figure 3 is a corresponding side view of Figure 1;
Figure 4 is a corresponding side view of Figure 2;
Figure 5 is an interrupted perspective view of a head of the machine of Figure 4.

### EMBIDOMENTS OF THE INVENTION.

With reference to the above-mentioned figures, 1 designates a multipurpose machine for monitoring and control of therapies, particularly regional cancer therapies in oncology.

The multipurpose machine 1, hereinafter briefly the machine 1, comprises a head 2 which has a box-like structure and is supported by a base 3 provided with means 4 for modifying the overall dimensions of the machine 1.

The head 2 is also provided with elements 5 for supporting adsorbent and/or filtering means, generally designated by 6, and with means 7 for controlling and regulating therapies, particularly chemotherapy and chemofiltration therapies.

The dimensions-modifying means 4 comprise a trolley 8 which is slidably received in the head 2, as better explained hereafter, and is provided with wheels 8A or the like, e.g. bearings or rollers, and arranged on the upper end of a vertical post 9 which rises from the base 3.

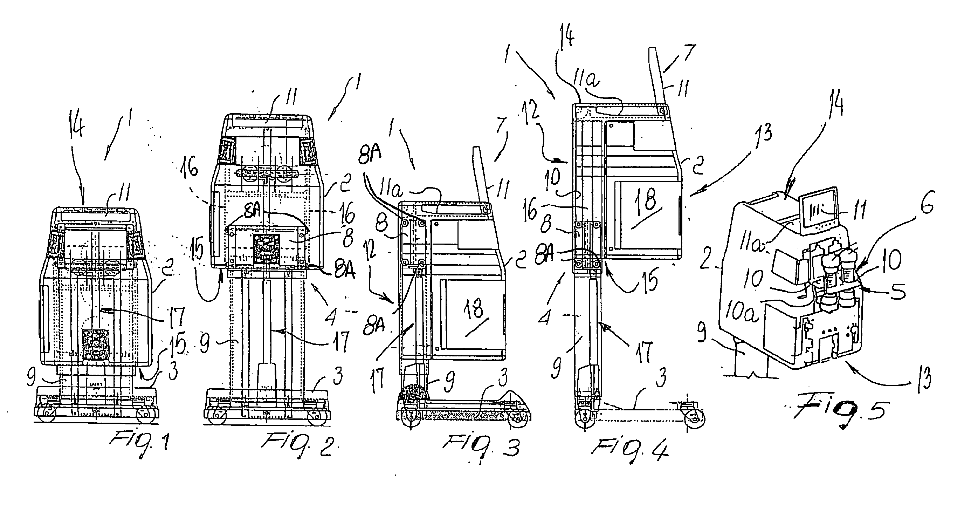

As it can be seen from Figure 3 and 4, the head 2 comprises a chamber 10 which is obtained in a portion of the latter which can be considered as the back 12 of the head 2 and which is opposite to a front portion 13.

The chamber 10 has a top zone 14 closed and a low zone 15 open, so that the trolley 8 can pass through it.

Inside the chamber 10 a couple of vertical and parallel rails 16 are fitted, along which the trolley 8 can slide.

According to the invention, a fluido-dynamic actuator 17 is arranged between the head 2 and the base 3, so that when it is activated, it can move the head 2 up and down in respect of the base 3, therefore modifying the height of the machine 1.

It is to be noted that the head 2 defines internally also a second chamber 18 which is adjacent to the chamber 10 and which is turned toward the front portion 13 of the machine 1, substantially cantilevered.

Inside the second chamber 18 are accommodated an emergency electronic circuit and one or more sets of duct circuits (not shown), which are provided with pumping means (also not shown) and connectable one another alternatively, in order to carry out therapies, e.g. the infusion of chemotherapeutics agents to a patient and to perform intraperitoneal hyperthermic perfusion therapies.

The supporting elements 5 are constituted by at least one plate 10 which is mounted so as to fold away in the front portion of the head 2 and provided with corresponding recesses 10a for accommodating and retaining packages 6, i.e the adsorbent and/or filtering means previously cited.

With reference to Figures 3, 4, 5, it is shown that the control and regulation means 7 are constituted by a controller, which is provided with a touch-screen monitor 11 which is arranged at the top of the head 2.

According to an embodiment of the machine 1, the monitor 11 can also be foldable back onto the head 2 into a fold-away position in a provided hollow seat 11a obtained for this purpose in the top zone 14.

The operation of the invention is as follows: when the head 2 is positioned downward as shown in Figures 1 and 3, it rest in a portable configuration or in a configuration in which it is adapted to be used besides a bed or an operating table, according to which its dimensions are reduced, namely its height.

Thus, the machine 1, in this configuration, is normally out of use and, if required, it is ready to be carried away.

For using the machine 1, the actuator 17 is activated and it pushes the head 2 upwardly, up to a selected height, in a working configuration.

To achieve the working configuration, the trolley 8 runs with wheels 8A along the parallel rails 16 and it slides from the inside of the chamber 10 toward the low open zone 15 of the head 2, as it can be seen in Figure 4.

Then the screen 11 is lifted from its seat 11a and the adsorbent and/or filtering means 6 are placed in the appropriate recesses 10a of holder plate 10, connected to the set of circuits accommodated inside the head 2.

The ends of the set of duct circuits are in their turn connected to the patient and the medical operator sets on the screen 11, by touching in each instance the surface thereof at the different displayed parameters, the preset values of these parameters in order to correctly perform the therapy, namely the chemotherapy session.

In practice, as soon as the therapeutic cycle begins, the machine 1 draws the blood from the patient, feeds into the extracorporeal circuits accommodated inside the head 2 and then returns it to the patient.

In practice, the administration of the solution that contains the chemotherapy agents again occurs under the constantly monitored control of the machine 1.

When the therapy (or a fixed number of therapies) is finished, the machine 1 can be set back into the portable configuration.

The actuator 17 pulls the head 2 downwardly and the trolley 8 slides along the parallel rails 16, returning inside the chamber 10.

The height of the machine 1 then decreases, as it can be seen in Figures 1 and 3, and the dimensions are thus reduced noticeably, this rendering the machine 1 portable and ready to be loaded on a car or an ambulance easily, or, alternatively, accommodated in a place of the operating theatre so that it cannot interferes with movements of medical staff, or furthermore to be adapted to the height of a bed on which a patient lies.

## Claims

1. A multipurpose machine for monitoring and control of therapies, comprising:
- a head (2) which has a box-like structure ;
- a base (3) to support said head (2); **characterized in that** modifying means (4) for modifying its dimensions are provided for.

2. A multipurpose machine according to claim 1, wherein said dimensions are at least the height.

3. The multipurpose machine according to claim 1, wherein said modifying means (4) comprises:
- a substantially vertical post (9), arranged between said head (2) and said (base (3);
- sliding means (8) arranged on said post (9) and slidably engageable with rails means (16) associated to said head (2) ;
- actuating means (17) suitable for moving said head (2) up and down, between a rest position and a working position, and vice versa.

4. The multipurpose machine according to claim 1 or 2, wherein said head (2) comprises a chamber (10) and a second chamber (18) placed adjacent each other.

5. The multipurpose machine according to claim 2, wherein said rails means are arranged inside said chamber (10).

6. The multipurpose machine according to claim 4, wherein this second chamber (18) accommodates at least a set of duct circuits and apparatus for carrying out therapies.

7. The multipurpose machine according to anyone of the preceding claims, wherein said head (2) is provided with supporting elements (5) suitable for supporting adsorbent and/or filtering means (6).

8. The multipurpose machine according to claim 7, wherein said supporting elements comprises at least one plate (10) which can be moved retractably in respect of said head (2) and is provided with corresponding recesses (10a) for accommodating said adsorbent and/or filtering means (6).
